# EUROPÄISCHE PATENTSCHRIFT

(11) **EP 0 880 344 B1**
(45) Veröffentlichungstag und Bekanntmachung des Hinweises auf die Patenterteilung: **06.08.2003**
(21) Anmeldenummer: 96941042.2
(22) Anmeldetag: 27.11.1996
(51) Int. Cl.: A61B 17/70

(54) **OSTEOSYNTHETISCHES BEFESTIGUNGSELEMENT**
OSTEOSYNTHETIC MOUNTING ELEMENT
ELEMENT DE FIXATION OSTEOSYNTHETIQUE

(30) Priorität: 15.02.1996 DE 19605640
(43) Veröffentlichungstag der Anmeldung: 02.12.1998
(73) Patentinhaber: Prof. Dr. med. Peter Griss, 35085 Ebsdorfergrund (DE)
(72) Erfinder: GRISS, Peter, D-35085 Ebsdorfergrund (DE); SCHMOTZER, Hans, CH-5000 Aarau (CH)
(74) Vertreter: Popp, Eugen, Dr.
(86) Internationale Anmeldenummer: EP9605243
(87) Internationale Veröffentlichungsnummer: WO97029707

(56) Entgegenhaltungen:
- EP-A- 0 487 895
- EP-A- 0 682 918
- DE-A- 4 425 357
- DE-C- 3 722 590

## Beschreibung

Die Erfindung betrifft ein osteosynthetisches Befestigungselement, insbesondere Pedikelschraube oder Wirbelsäulenhaken, gemäß dem Oberbegriff des Anspruches 1 bzw. gemäß dem Oberbegriff des Anspruches 8.

Zur Behandlung von Wirbelsäulenverletzungen sind verschiedene Methoden und Osteosynthese-Vorrichtungen bekannt. Aus der FR-A-2 642 643 ist ein Implantat zur Wirbelsäulenkorrektur bekannt mit einer Längsachse, einem ersten, am Knochen verankerbaren Abschnitt, einem in Richtung der Längsachse sich an den ersten Abschnitt anschließenden zweiten Abschnitt, der einen durch zwei seitliche Schenkel begrenzten Durchgang zur Aufnahme eines stabförmigen Verbindungselements umfaßt, einem koaxialen Innenzylnder zur Einführung zwischen die erwähnten Schenkel, wobei die äußere Umfangsfläche des Innenzylinders ein mit einem an der Innenfläche der beiden Schenkel ausgebildeten Innengewinde korrespondierendes Außengewinde aufweist, sowie einem Kappenteil mit koaxialer äußerer Krempe zur Umschließung der Schenkel. Gemäß einer Weiterbildung dieses Implantats ist in der EP-B 0 496 851 vorgeschlagen, den koaxialen Innenzylinder und den Kappenteil als einstückiges Klemmteil für das stabartige Verbindungselement auszubilden.

Die vorgenannten Konstruktionen haben den Nachteil, daß das stabartige Verbindungselement zwischen den beiden Schenkeln des zweiten Abschnitts nur in Richtung senkrecht zur Längsachse des Implantats fixierbar ist.

Zur Behebung des letztgenannten Nachteils gibt es eine Vielzahl von konstruktiven Vorschlägen, z. B. das Implantat gemäß der EP-B 0 408 489, EP-A 0 572 790, DE-A 43 30 837 oder EP-B 0 487 895. All diesen Vorschlägen ist gemeinsam, daß das stabartige Verbindungselement jeweils zwischen den beiden Schenkeln des zweiten Implantatabschnitts mittels verschwenkbar gelagerter Klemmkörper fixiert wird. Da der Raum zwischen den beiden Schenkeln begrenzt ist, sind die bekannten Konstruktionen entsprechend diffizil ausgebildet und hinsichtlich ihrer Bruchfestigkeit äußerst fraglich. Auch zeichnen sich die bekannten Konstruktionen durch eine Vielzahl einzeln zu handhabender Bauelemente aus mit der Gefahr, daß das eine oder andere Bauelement bei der Implantation vergessen wird. Ein solches Mißgeschick kann fatale Folgen haben. Dies ist auch der Grund, warum bei dem Implantat gemäß der EP-B 0 496 851 auf eine einstückige Ausbildung des zentralen und als Klemmkörper dienenden Innenzylinders mit dem die beiden Schenkel umschließenden Kappenteil Wert gelegt wird. Nur - wie gesagt - erlaubt diese bekannte Konstruktion keine Einstellung des stabartigen Verbindungselements in einem von 90° zur Längsachse abweichenden Winkel.

In der EP-A 0 487 895 ist eine Verankerungseinrichtung beschrieben, die aus einer Pedikelschraube mit einem U-förmigen Aufnahmeteil besteht. Im Aufnahmeteil ist durch eine Auflage eine Rotationsachse festgelegt, um welche eine Stab schwenkbar einlegbar ist. Auf dem Stab befindet sich formschlüssig gelagert ein Formteil, das eine Fläche besitzt, die , wenn man das Formteil an die zwangsläufig vorgegebene, richtige Stelle schiebt, einen Radius zur Rotationsachse besitzt. Eine Mutter wird an Gewindeteilen an den Backen des U-förmigen Aufnahmeteils eingedreht und bringt mit ihrem unteren Rand zunächst das Formteil in die zwangsläufig richtige Stellung zur Achse und fixiert bei weiterem Zudrehen den Stab und die Pedikelschraube, sodaß keine unerwünschten Spannungen entstehen. Die Einrichtung findet in der Osteosynthese, insbesondere zur Stabilisierung von Wirbelsäulensegmenten, Verwendung.

In der EP-A 0 682 918 ist eine osteosynthetische Befestigungsvorrichtung für die Wirbelsäule beschrieben. Diese besteht aus einem am Knochen verankerbaren Abschnitt und einem weiteren Abschnitt mit zwei seitlichen parallelen Schenkeln zur Aufnahme eines stabartigen Verbindungselements. Weiterhin weist die Vorrichtung einen Deckel mit einem Rand und einem Gewindeabschnitt auf, wobei der Rand über die beiden Schenkel vorsteht und der Gewindeabschnitt zwischen die beiden Schenkel einschraubbar ist. Zwischen dem Deckel und dem stabartigen Verbindungselement befindet sich ein Klemmelement, das gegen das stabartige Verbindungselement drückbar ist. Das Klemmelement hat eine dreidimensional ausgebildete Radialnut zur Aufnahme des stabartigen Verbindungselements.

Hinsichtlich der Handhabung und der Gefahr, kein Bauteil bei der Implantation zu vergessen, hat sich die Konstruktion gemäß der EP-A 0 465 158 als besonders vorteilhaft erwiesen. Diese Konstruktion ist dadurch gekennzeichnet, daß sie einen über den äußeren Rand der beiden Schenkel des zweiten Implantatabschnitts vorstehenden Deckel mit einem zentralen, zwischen die beiden Schenkel einschraubbaren Gewindeabschnitt aufweist, sowie einen mit radialem Spiel sich um die beiden Schenkel herum erstreckenden Klemmring umfaßt, der zur klemmenden Fixierung des Verbindungselements innerhalb des im zweiten Abschnitt ausgebildeten Durchgangs durch den Deckel bzw. dessen äußeren Umfangsrand gegen das Verbindungselement drückbar ist. Natürlich muß der zentrale Gewindeabschnitt so kurz bemessen sein, daß er in Klemmstellung des äußeren Klemmrings vom stabartigen Verbindungselement ausreichend beabstandet ist, d. h. auf diese nicht einwirkt. Die Klemmung des stabartigen Verbindungselements erfolgt ausschließlich über den äußeren Klemmring. Der Klemmring kann aufgrund der vorgenannten Dimensionierung des zwischen die beiden Schenkel des zweiten Implantatabschnitts einschraubbaren Gewindeabschnitts nicht vergessen werden; denn dann würde überhaupt keine Klemmung des stabartigen Verbindungselements stattfinden. Natürlich ist der zentrale Gewindeabschnitt auch so kurz bemessen, daß selbst bei fehlendem Klemmring und vollständiger Einschraubung des Deckels keine Einwirkung des Gewindeabschnitts auf das stabartige Verbindungselement stattfindet. Ohne Klemmring würde das stabartige Verbindungselement also lose im Durchgang zwischen den beiden Schenkeln des zweiten Implantatabschnitts gehalten sein.

Nachteilig ist bei der letztgenannten Konstruktion jedoch, daß auch hier das stabartige Verbindungselement nur in einem Winkel senkrecht zur Längsachse des osteosynthetischen Befestigungselements fixierbar ist. Die Einsatzmöglichkeiten des bekannten Befestigungselements sind also dementsprechend beschränkt.

Der vorliegenden Erfindung liegt die Aufgabe zugrunde, ein osteosynthetisches Befestigungselement zu schaffen, welches äußerst sicher in der Handhabung ist, aus einem Minimum von Bauteilen besteht und eine klemmende Fixierung des zugeordneten stabartigen Verbindungselements in einem vom 90° zur Längsachse des Befestigungselements abweichenden Winkel erlaubt, und zwar sowohl in einer Ebene parallel zur Durchgangsebene als auch senkrecht dazu.

Diese Aufgabe wird in einer ersten Ausführungsform durch die kennzeichnenden Merkmale des Anspruches 1 und in einer zweiten Ausführungsform durch die kennzeichnenden Merkmale des Anspruches 8 gelöst, wobei es sich bei der Konstruktion nach Anspruch 1 um eine sogenannte "äußere Klemmfixierung" des stabartigen Verbindungselements handelt, während die Konstruktion nach Anspruch 8 eine sogenannte "innere Klemmfixierung" des stabartigen Verbindungselements definiert.

Konstruktive Details der beiden vorgenannten Alternativlösungen sind in den Ansprüchen 2 bis 7 bzw. 9 und 10 beschrieben.

Die erfindungsgemäßen Konstruktionen haben den Vorteil, daß mit einem Minimum von Bauteilen, die zur klemmenden Fixierung des stabartigen Verbindungselements zwingend notwendig sind und damit beim Gebrauch nicht vergessen werden können, eine klemmende Fixierung des stabartigen Verbindungselements auch in einem Winkel abweichend von 90° zur Längsachse des osteosynthetischen Befestigungselements möglich ist. Beide Ausführungsformen gestatten ein Verschwenken des stabartigen Verbindungselements in einer Ebene parallel zu der durch den kanalartigen Durchgang definierten (Durchgangs-)Ebene sowie in einer Ebene senkrecht dazu, sofern die Breite des Durchgangs größer ist als die maximale Breite bzw. der Durchmesser des stabartigen Verbindungselements.

Nachstehend werden anhand der beigefügten Zeichnung zwei bevorzugte Ausführungsbeispiele erfindungsgemäß ausgebildeter Befestigungselemente näher erläutert. Es zeigen:
- Fig. 1: einen Längsschnitt durch den zweiten bzw. oberen Abschnitt (Kopf) einer Pedikelschraube parallel zur Durchgangsebene und in vergrößertem Maßstab;
- Fig. 2: den oberen Abschnitt (Kopf) der Pedikelschraube samt stabartigem Verbindungselement gemäß Fig. 1 in perspektivischer Draufsicht;
- Fig. 3: die Pedikelschraube gemäß Fig. 2 in perspektivischer Schrägansicht von der Seite;
- Fig. 4: den zweiten bzw. oberen Abschnitt (Kopf) einer zweiten Ausführungsform einer Pedikelschraube gemäß Erfindung teilweise im Längsschnitt senkrecht zur Durchgangsebene, teilweise in Ansicht; und
- Fig. 5: einen Teil des oberen bzw. zweiten Abschnitts (Kopf) der Pedikelschraube gemäß Fig. 4 in Seitenansicht, wobei die Pedikelschraube gegenüber der Darstellung in Fig. 4 um 90° verdreht ist.

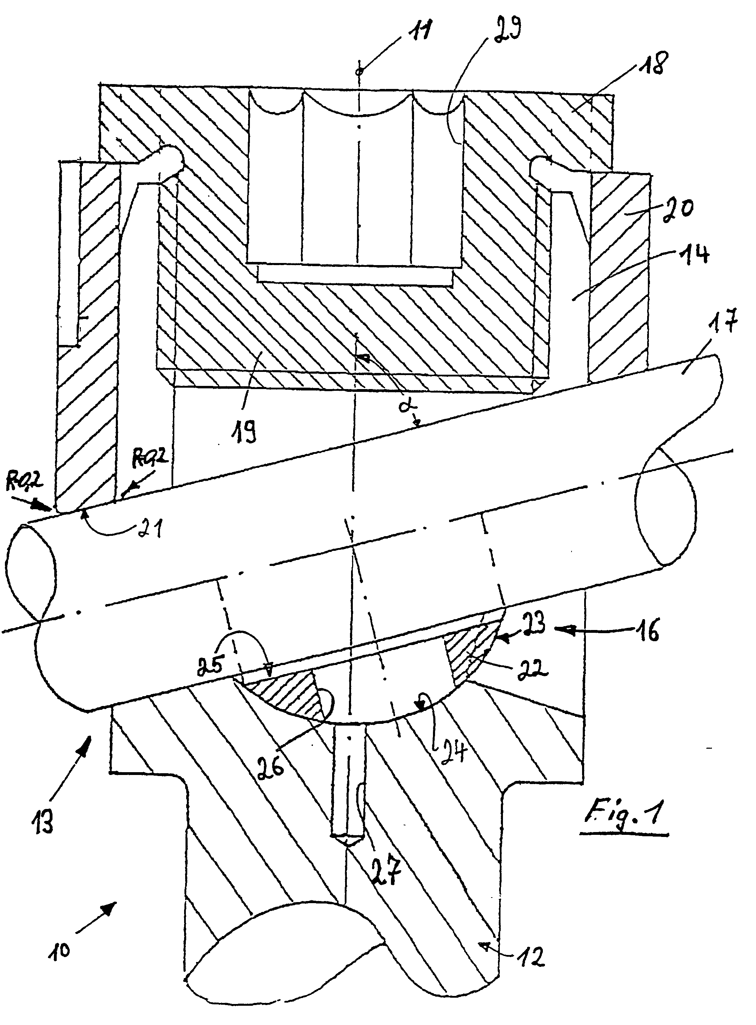

In den Fig. 1 bis 3 ist ein osteosynthetisches Befestigungsmittel in Form einer Pedikelschraube 10 dargestellt, die folgende Merkmale umfaßt:
- eine Längsachse 11;
- einen ersten, unteren am Knochen verankerbaren Abschnitt 12 in Form eines nicht näher dargestellten Schraubabschnitts;
- einen in Richtung der Längsachse 11 sich an den ersten bzw. unteren Abschnitt 12 anschließenden zweiten oberen Abschnitt 13, der den Kopf der Pedikelschraube 10 definiert und der einen durch zwei seitliche Schenkel kanalartig begrenzten Durchgang 16 zur Aufnahme eines stabartigen Verbindungselements 17 umfaßt;
   einen über den äußeren Rand der beiden Schenkel 14, 15 vorstehenden Deckel 18 mit einem zentralen, zwischen die beiden Schenkel 14, 15 einschraubbaren Gewindeabschnitt 19; und
- einen mit geringem radialen Spiel sich um die beiden Schenkel 14, 15 herum erstreckenden Klemmring 20, der zur klemmenden Fixierung des Verbindungselements 17 innerhalb des im zweiten oberen Abschnitt 13 ausgebildeten Durchgangs 16 durch den Deckel 18 bzw. dessen äußeren Umfangsrand gegen das Verbindungselement 17 drückbar ist, und zwar mit seinem unteren stirnseitigen Umfangsrand 21.

Der dem stabartigen Verbindungselement 17 zugewandte stirnseitige Umfangsrand 21 des Klemmrings 20 erstreckt sich in einer schräg zur Längsachse 11 liegenden Ebene. Der entsprechende Winkel α zwischen dieser Ebene und der Längsachse 11 des Befestigungselements, welche im montierten Zustand mit der Längsachse des Klemmrings 20 fluchtet, beträgt zwischen etwa 70 bis 85° (siehe Fig. 1). Der Klemmring 20 ist also an der dem Verbindungselement 17 zugewandten Seite schräg angeschnitten. Des weiteren läßt sich der Klemmring im montierten Zustand und nach Plazierung des stabartigen Verbindungselements 17 im Durchgang 16 um die Längsachse 17 in jede gewünschte Lage drehen. Dementsprechend ändert sich auch die Neigung des stabartigen Verbindungselements 17 relativ zur Längsachse 11, und zwar insbesondere in einer Ebene parallel zu der durch den Durchgang 16 definierten Ebene, so wie dies in Fig. 1 sehr deutlich dargestellt ist. Sobald die gewünschte Lage des stabartigen Verbindungselements 17 innerhalb des Durchgangs 16 der Pedikelschraube 10 eingestellt ist, wird diese Lage durch den Deckel 18 über den Klemmring 20 fixiert. Zu diesem Zweck wird der Deckel 18 mit seinem Gewindeabschnitt 19 zwischen die beiden Schenkel 14, 15 eingeschraubt, wobei die minimale Höhe der Klemmhülse 20 größer ist als der Abstand zwischen der Unterseite des Gewindeabschnitts 19 und der Unterseite des Deckels 18. Damit ist gewährleistet, daß die Unterseite des Gewindeabschnitts 19 stets ausreichend von der Oberseite des stabartigen Verbindungselements 17 beabstandet ist, und zwar auch in der maximalen Schwenklage des Verbindungselements 17 relativ zur Längsachse 11.

Am Boden des Durchgangs 16 ist ein universal gelenkig gelagertes Stützelement 22 für das stabartige Verbindungselement 17 angeordnet derart, daß das Verbindungselement 17 innerhalb des Durchgangs 16 sowohl in einer Ebene parallel zu der durch den Durchgang 16 definierten Ebene als auch in einer Ebene quer dazu verschwenkbar und mitteils des Klemmrings 20 in einer bevorzugten Schwenklage fixierbar ist. Konkret weist das Stützelager 22 eine schalen- bzw. kugelsegmentartige Lagerfläche 23 auf, die mit einer entsprechenden Stützfläche 24 am Boden des Durchgangs 16 korrespondiert. An der dem stabartigen Verbindungselement 17 zugewandten Seite ist das Stützelement 22 mit einer Aufnahmenut 25 zur Aufnahme des stabartigen Verbindungselements 17 versehen, wobei bei einem Verbindungselement 17 mit kreisförmigem Querschnitt die Aufnahmenut 25 einen etwa halbkreisförmigen Querschnitt aufweist. In der Oberfläche der Aufnahmenut 25 können Querrippen, insbesondere ein Feingewinde eingearbeitet sein, um eine noch bessere Fixierung des stabartigen Verbindungselements 17 innerhalb des Durchgangs 16 zu erhalten. Das Stützelement 22 ist vorzugsweise am Boden des Durchgangs 16 gehalten derart, daß es nach Montage nicht verlorengehen kann. Das Stützelement 22 ist also Teil der Pedikelschraube 10. Konkret erfolgt die Fixierung des Stützelements 22 am Boden des Durchgangs 16 durch einen über die Stützfläche 24 vorstehenden Stift, der mit geringer Pressung in einer an der der Stützfläche 24 zugewandten Lagerfläche 23 des Stützelements ausgebildeten, insbesondere durchgehend ausgebildeten Aufnahmenut 26 gehalten ist, wobei sich die Aufnahmenut 26 parallel zur Aufnahmenut 25 für das stabartige Verbindungselement 17 erstreckt. Der erwähnte Haltestift ist in Fig. 1 nicht näher dargestellt. Es ist lediglich die Aufnahmebohrung für den Haltestift in der Stützfläche 24 gezeigt und mit der Bezugsziffer 27 gekennzeichnet. Im übrigen wird bezüglich des Stützelements 22 und dessen Fixierung am Boden des Durchgangs 16 auf die WO 94/14 384 verwiesen, die auf die Anmelderin zurückgeht.

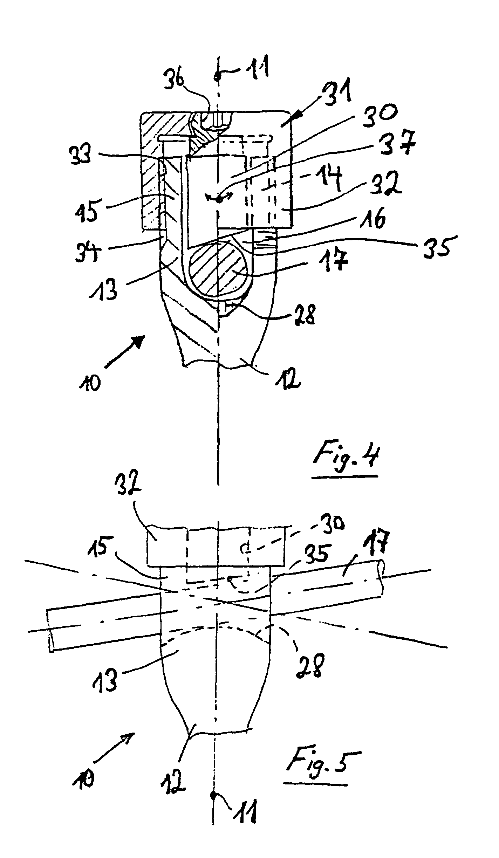

Alternativ kann statt des schwenkbar gelagerten Stützelements 22 am Boden des Durchgangs 16 eine sattelartige Erhebung ausgebildet sein, wie sie bei dem Ausführungsbeispiel nach den Fig. 4 und 5 dargestellt und mit der Bezugsziffer 28 gekennzeichnet ist. Über diese sattelartige Erhebung ist das stabartige Verbindungselement 17 in eine bevorzugte Schwenklage relativ zur Längsachse 11 des Befestigungselements 10 bringbar und bei Verwendung eines Klemmrings 20 in dieser mittels desselben fixierbar.

An der Oberseite des Deckels 18 ist ein Innensechskant 29 ausgebildet. Dieser dient zum Eingriff eines entsprechenden Drehwerkzeuges für den Deckel 18.

Um den Klemmring 20 in die gewünschte Drehlage zu bringen, ist die äußere Umfangsfläche des Klemmrings 20 gerändelt oder anderweitig strukturiert, z. B. mit Flachseiten zum Angriff eines Maulschlüssels oder mit axialen Ausnehmungen zum Angriff eines komplementären Drehwerkzeuges versehen. Die erwähnten Flachseiten und/oder Ausnehmungen dienen auch zum erleichterten Drehen des Klemmrings 20 zwischen zwei Fingern des Operateurs.

Der dem stabartigen Verbindungselement 17 zugewandte stirnseitige Umfangsrand 21 des Klemmrings 20 weist vorzugsweise abgerundete Umfangskanten (R = 0,2 mm in Fig. 1) auf, so daß ein Eingraben des Klemmrings 20 in das stabartige Verbindungselement 17 vermieden wird, wodurch die Oberfläche desselben in unzulässiger Weise zerstört würde.

Um eine noch bessere Klemmung ohne plastische Verformung des stabartigen Verbindungselements 17 durch den Klemmring 20 zu erreichen, kann der dem Verbindungselement zugewandte stirnseitige Umfangsrand 21 mehrere gleichmäßig diametral zur Längsachse 11 über den Umfang verteilt angeordnete Ausnehmungen aufweisen, deren axiale Begrenzung jeweils dem zugewandten Umfang des stabartigen Verbindungselements 17 angepaßt ist. Der Umfangsrand 21 weist also bei dieser Ausführungsform kreisbogenförmige Ausschnitte auf, die der Längsachse 11 diametral zueinander angeordnet und gleichmäßig über den Umfangsrand 21 verteilt sind. Damit wird das stabartige Verbindungselement 17 sowohl von oben als auch von unten umgriffen und zwischen dem Klemmring 20 und dem Stützelement 22 regelrecht eingebettet. Die erwähnten Randausnehmungen verhindern auch eine unerwünschte Mitnahme bzw. Verdrehung des Klemmrings 20 beim Einschrauben des Deckels 18 durch diesen.

Das stabartige Verbindungselement 17 kann als starrer oder plastisch verbiegbarer Stab oder nach Art eines flexiblen Kabels ausgebildet sein. In. jedem Fall ist der beschriebene Klemmechanismus einsetzbar.

Um die Handhabung der beschriebenen Konstruktion zusätzlich zu erleichtern, kann der Klemmring 20 drehbar am äußeren Umfangsrand des Deckels 18 gelagert sein derart, daß der Deckel 18 und Klemmring 20 eine Baueinheit bilden. In diesem Fall besteht die gesamt Anordnung aus nur insgesamt drei Teilen, nämlich der Pedikelschraube 10, Deckel und Klemmring 18, 20 sowie Verbindungselement 17.

Die vorgenannten Teile bestehen vorzugsweise jeweils aus Titan bzw. einer Titanlegierung.

Durch das Stützlager 22 und den Klemmring 20 soll eine Kippung des stabartigen Verbindungselements in der Ebene parallel zur Durchgangsebene um ± 15° möglich sein. In einer Ebene senkrecht dazu ist eine Verdrehmöglichkeit um ± 5° vorteilhaft.

Der Klemmring 20 hat eine weitere Funktion; er verhindert nämlich eine unzulässige radiale Spreizung der beiden Schenkel 14, 15 nach außen beim Einschrauben des Deckels 18.

Die Ausführungsform einer Pedikelschraube gemäß den Fig. 4 und 5 ist durch folgende Merkmale gekennzeichnet:
- eine Längsachse 11;
- einen ersten, unteren und am Knochen verankerbaren Schraub- oder Hakenabschnitt 12;
- einen in Richtung der Längsachse 11 sich an den ersten unteren Abschnitt 12 anschließenden zweiten oberen Abschnitt 13, der einen durch zwei seitliche Schenkel 14, 15 begrenzten Durchgang 16 zur Aufnahme eines stabartigen Verbindungselements 17 umfaßt;
- ein zwischen den beiden Schenkeln 14, 15 einbringbares Klemmelement 30 zur klemmenden Fixierung des stabartigen Verbindungselements 17 innerhalb des Durchgangs 16; und
- eine über die beiden Schenkel 14, 15 schraubbare Kappe 31, deren äußere Krempe 32 die beiden Schenkel 14, 15 umschließt und die mit dem als Zylinderkörper ausgebildeten Klemmelement 30 verbunden ist.

Die Krempe 32 der Kappe 31 weist ein Innengewinde 33 auf, welches mit einem an der äußeren Umfangsfläche der beiden Schenkel 14, 15 des zweiten oberen Abschnitts 13 ausgebildeten Außengewinde 34 korrespondiert. Das Klemmelement 30 ist am Deckel der Kappe 31 relativ zur Längsachse 11 des Befestigungselements 10 verdrehbar gelagert, wobei es von oben her durch den Deckel 18 hindurch zur Einstellung einer gewünschten Drehlage zugänglich ist. Die dem Verbindungselement 17 zugewandte und mit diesem zusammenwirkende Stirnseite 35 erstreckt sich schräg zur Längsachse 11 des Befestigungselements 10. Auf diese Weise kann durch Verdrehen des Klemmelements 30 um die Längsachse 11 die Kipplage des stabartigen Verbindungselements 17 in einer Ebene parallel zur Durchgangsebene eingestellt und durch Festschrauben der Kappe 31 fixiert werden. Sofern der Durchgang 16 ausreichend breit gestaltet wird, ist auch ein Verdrehen des stabartigen Verbindungselements 17 um eine Ebene quer zur Durchgangsebene möglich, wobei der Verdrehwinkel vorzugsweise wieder ± 5° beträgt.

Bei der dargestellten Ausführungsform ist das stabartige Verbindungselement 17 am Boden des Durchgangs 16 um eine sattelartige Erhebung 28 kippbar. Stattdessen kann am Boden des Durchgangs 16 ein Stützelement entsprechend dem Stützelement 22, wie es anhand der Fig. 1 beschrieben ist, angeordnet sein.

Das Klemmelement 30 verhindert bei entsprechender Dimensionierung ein radiales Ausweichen der beiden Schenkel 14, 15 nach innen, wenn die Schraubkappe 31 aufgeschraubt und unter vorbestimmtem Drehmoment angezogen wird. Das Klemmelement 30 ist vorzugsweise unlösbar am Deckel der Kappe 31 drehgelagert. Es ist jedoch auch denkbar, das Klemmelement 30 als gesondertes Bauteil auszuführen und vor dem Aufschrauben der Kappe 31 in den Raum zwischen die beiden Schenkel 14, 15 einzubringen und in der gewünschten Drehlage zu fixieren.

Bei der dargestellten Ausführungsform ist das Klemmelement 30 so im Deckel der Kappe 31 drehgelagert, daß es unmittelbar von außen her zugänglich ist. Dementsprechend weist das Klemmelement 30 an seiner dem Verbindungselement 17 abgewandten Stirnseite einen Innensechskant 36 auf, über den das Verbindungselement 30 bei schon aufgeschraubter Kappe 31 um die Längsachse 11 verdreht werden kann in eine Stellung, die einer gewünschten Kipplage des stabartigen Verbindungselements 17 entspricht. Die Verdrehbarkeit des Klemmelements 30, dessen Umfangsfläche vorzugsweise glatt ausgebildet ist, um die Längsachse 11, ist in Fig. 4 mit dem Doppelpfeil 37 angedeutet.

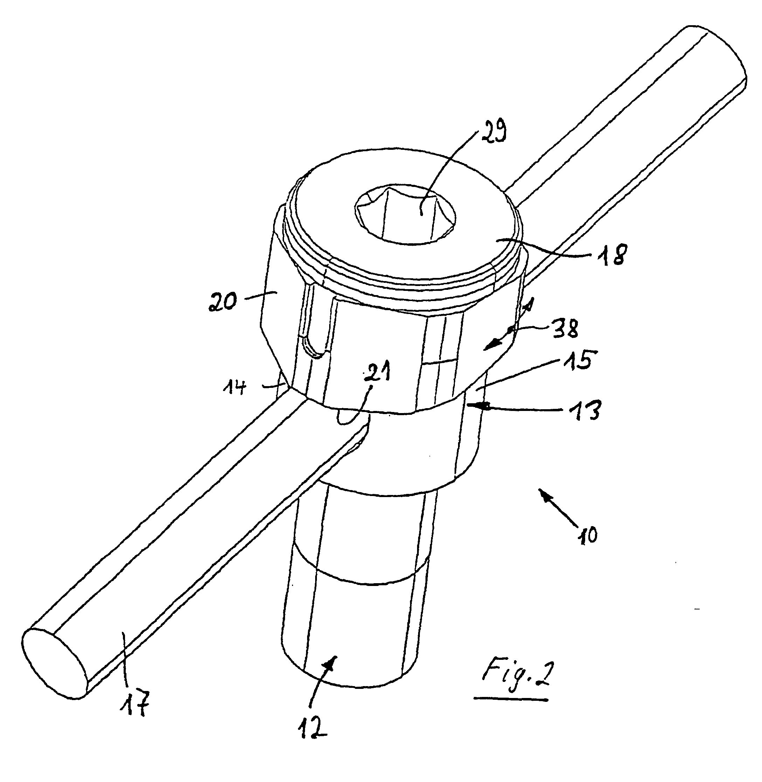

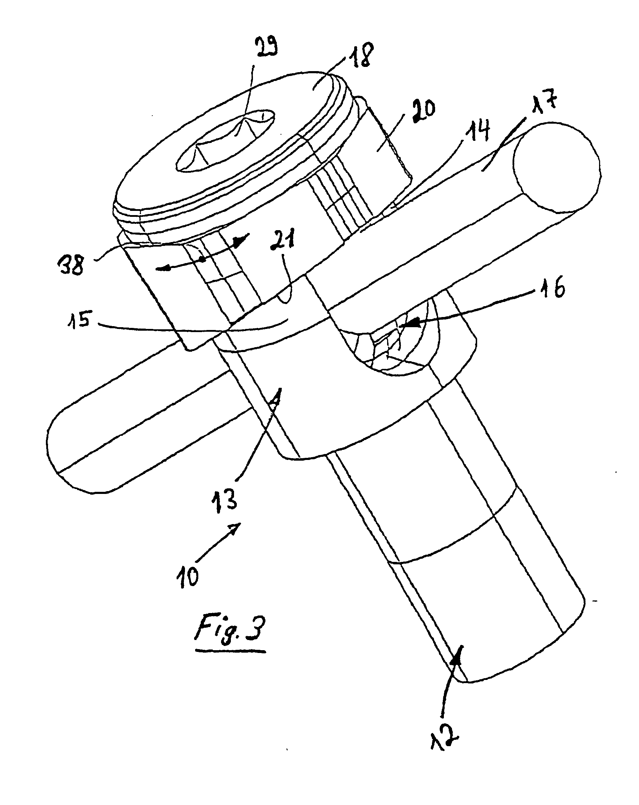

In den Fig. 2 und 3 ist die entsprechende Verdrehbarkeit des Klemmrings 20 um die Längsachse 11 mit dem Doppelpfeil 38 angedeutet.

Vor dem endgültigen Festklemmen des Verbindungselements 17 innerhalb des Durchgangs 16 ist das Verbindungselement 17 sowohl kippbar als auch in Längsrichtung verschiebbar oder um die Längsachse verdrehbar. Diese Freiheitsgrade werden durch die beschriebenen Klemmechanismen blockiert.

### Bezugszeichenliste

- 10: Pedikelschraube
- 11: Längsachse
- 12: erster (unterer) Abschnitt
- 13: zweiter (oberer) Abschnitt
- 14: Schenkel
- 15: Schenkel
- 16: Durchgang
- 17: stabartiges Verbindungselement
- 18: Deckel
- 19: Gewindeabschnitt
- 20: Klemmring
- 21: Umfangsrand
- 22: Stützelement
- 23: Lagerfläche
- 24: Stützfläche
- 25: Aufnahmenut
- 26: Aufnahmenut
- 27: Aufnahmebohrung
- 28: sattelartige Erhebung
- 29: Innensechskant
- 30: Klemmelement
- 31: Kappe
- 32: Krempe
- 33: Innengewinde
- 34: Außengewinde
- 35: Stirnseite
- 36: Innensechskant
- 37: Doppelpfeil
- 38: Doppelpfeil

## Patentansprüche

1. Osteosynthetisches Befestigungselement, insbesondere Pedikelschraube (10) oder Wirbelsäulenhaken, mit
a) einer Längsachse (11),
b) einem ersten, am Knochen verankerbaren Abschnitt (12)
c) einem in Richtung der Längsachse (11) sich an den ersten Abschnitt (12) anschließenden zweiten Abschnitt (13), der einen durch zwei seitliche Schenkel (14, 15) begrenzten Durchgang (16) zur Aufnahme eines stabartigen Verbindungselements (17) umfaßt,
d) einem über den äußeren Rand der beiden Schenkel (14, 15) vorstehenden Deckel (18) mit einem zentralen, zwischen die beiden Schenkel (14, 15) einschraubbaren Gewindeabschnitt (19), und mit
e) einem mit radialem Spiel sich um die beiden Schenkel (14, 15) herumerstreckenden Klemmring (20), der zur klemmenden Fixierung des stabartigen Verbindungselements (17) innerhalb des im zweiten Abschnitt (13) ausgebildeten Durchgangs (16) durch den Deckel (18) bzw. dessen äußeren Umfangsrand (21) gegen das Verbindungselement (17) drückbar ist, wobei der dem Verbindungselement (17) zugewandte äußere Umfangsrand (21) sich durchgehend in einer Ebene erstreckt, oder mehrere gleichmäßig diametral zur Längsachse (11) über den Umfang verteilt angeordnete Ausnehmungen aufweist, deren axiale Begrenzung jeweils dem zugewandten Umfang des stabartigen Verbindungselements (17) angepaßt ist.
**dadurch gekennzeichnet, daß**
die vom äußeren Umfangsrand (21) definierte Ebene schräg (Winkel α) zur Längsachse (11) geneigt ist.

2. Befestigungselement nach Anspruch 1,
**dadurch gekennzeichnet, daß**
am Boden des Durchgangs (16) im zweiten Abschnitt (13) des Befestigungselements (10) ein universal gelenkig gelagertes Stützelement (22) für das stabartige Verbindungselement (17) angeordnet ist, so daß das Verbindungselement (17) innerhalb des Durchgangs (16) sowohl in einer Ebene parallel zu der durch den Durchgang (16) definierten Ebene als auch in einer Ebene quer dazu verschwenkbar und mittels des Klemmrings (20) in einer bevorzugten Kipp- und Drehlage fixierbar ist.

3. Befestigungselement nach Anspruch 1,
**dadurch gekennzeichnet, daß**
am Boden des im zweiten Abschnitt (13) des Befestigungselements (10) ausgebildeten Durchgangs (16) eine sattelartige Erhebung (28) ausgebildet ist, über die das stabartige Verbindungselement (17) in eine bevorzugte Kipplage relativ zur Längsachse (11) des Befestigungselements (10) bringbar und in dieser Lage mittels des Klemmrings (20) fixierbar ist.

4. Befestigungselement nach einem der Ansprüche 1 bis 3,
**dadurch gekennzeichnet, daß**
zum Zwecke eines erleichterten Drehens des Klemmrings (20) um die Längsachse (11) die äußere Umfangsfläche des Klemmrings (20) strukturiert, insbesondere gerändelt ist.

5. Befestigungselement nach einem der Ansprüche 1 bis 4,
**dadurch gekennzeichnet, daß**
der dem Verbindungselement (17) zugewandte, stirnseitige Umfangsrand (21) des Klemmrings (20) abgerundete Umfangskanten (R) aufweist.

6. Befestigungselement nach einem der Ansprüche 1 bis 5,
**dadurch gekennzeichnet, daß**
das stabartige Verbindungselement (17) als starrer oder plastisch verbiegbarer Stab, oder nach Art eines flexiblen Kabels ausgebildet ist.

7. Befestigungselement nach einem der Ansprüche 1 bis 6,
**dadurch gekennzeichnet, daß**
der Klemmring (20) drehbar am äußeren Umfangsrand des Deckels (18) gelagert ist derart, daß Deckel (18) und Klemmring (20) eine Baueinheit bilden.

8. Osteosynthetisches Befestigungselement, insbesondere Pedikelschraube (10) oder Wirbelsäulenhaken, mit
a) einer Längsachse (11),
b) einem ersten, am Knochen verankerbaren Abschnitt (12),
c) einem in Richtung der Längsachse (11) sich an den ersten Abschnitt (12) anschließenden zweiten Abschnitt (13), der einen durch zwei seitliche Schenkel (14, 15) begrenzten Durchgang (16) zur Aufnahme eines stabartigen Verbindungselements (17) umfaßt,
d) einem zwischen die beiden Schenkeln (14, 15) einbringbaren Klemmelement (30) zur klemmenden Fixierung des stabartigen Verbindungselements (17) innerhalb des im zweiten Abschnitt (13) des Befestigungselements (10) ausgebildeten Durchgangs (16), und mit
e) einer am zweiten Abschnitt (13) des Befestigungseiements (10) verschraubbaren Kappe (31), deren äußere Krempe (32) die beiden Schenkel (14, 15) des zweiten Abschnitts (13) umschließt und die mit dem Klemmelement (30) verbunden ist,
wobei die Krempe (32) der Kappe (31) ein Innengewinde (33) aufweist, welches mit einem an der äußeren Umfangsfläche der beiden Schenkel (14, 15) des zweiten Abschnitts (13) ausgebildeten Außengewinde (34) korrespondiert,
**dadurch gekennzeichnet, daß**
das Klemmelement (30) am Deckel der Kappe (31) relativ zur Längsachse (11) verdrehbar gelagert ist und
daß die dem Verbindungselement (17) zugewandte und mit diesem zusammenwirkende Stirnseite (35) eben ausgebildet ist und sich schräg zur Längsachse (11) erstreckt.

9. Befestigungselement nach Anspruch 8,
**dadurch gekennzeichnet, daß**
das Klemmelement (30) zur Einstellung einer gewünschten Drehlage durch den Deckel der Kappe (31) hindurch von außen her zugänglich ist.

10. Befestigungselement nach Anspruch 7 oder 8,
**dadurch gekennzeichnet, daß**
das Klemmelement (30) als an der dem stabartigen Verbindungselement (17) zugewandten Stirnseite schräg angeschnittener Zylinderabschnitt mit glatter Umfangsfläche ausgebildet ist.

## Claims

1. An osteosynthetic fixation element, more particularly a pedicle screw (10) or a spine hook, comprising
a) a longitudinal axis (11),
b) a first portion (12) anchorable to the bone,
c) a second portion (13), adjoining the first portion (12) in the direction of the longitudinal axis (11), which comprises a passage (16) defined by two lateral arms (14, 15) for receiving a rod-like connection element (17),
d) a cover (18) projecting over the exterior edge of the two arms (14, 15), comprising a central threaded portion (19) adapted for screwing into place between the two arms (14, 15), and comprising
e) a clamping ring (20) extending around the two arms (14, 15) with radial clearance, which, in order to fix the rod-like connection element (17) to effect the clamping thereof within the passage (16) formed in the second portion (13), is adapted for pressing against the connection element (17) by means of the cover (18) or the exterior peripheral edge (21) thereof, wherein the exterior peripheral edge (21) directed towards the connection element (17) extends continuously in a plane, or has several recesses arranged so as to be evenly distributed over the periphery diametrically to the longitudinal axis (11), the axial boundary of the said recesses in each case being adapted to the periphery, directed towards them, of the rod-like connection element (17),
**characterised in that**
the plane defined by the exterior peripheral edge (21) is inclined obliquely
(angle α) to the longitudinal axis (11).

2. A fixation element according to Claim 1,
**characterised in that**
a support element (22), mounted for universal articulation, for the rod-like connection element (17) is arranged on the base of the passage (16) in the second portion (13) of the fixation element (10), so that the connection element (17) is pivotable within the passage (16) both in a plane parallel to the plane defined by the passage (16) and in a plane transversely thereto and is fixable in a preferred tilt- and rotational position by means of the clamping ring (20).

3. A fixation clement according to Claim 1,
**characterised in that**
a saddle-like elevation (28) is formed on the base of the passage (16) formed in the second portion (13) of the fixation element (10), via which the rod-like connection element (17) is movable into a preferred tilt position relative to the longitudinal axis (11) of the fixation element (10) and is fixable in this position by means of the clamping ring (20).

4. A fixation element according to one of Claims 1 to 3,
**characterised in that**
the exterior peripheral surface of the clamping ring (20) is textured, more particularly knurled, for the purpose of making turning the clamping ring (20) around the longitudinal axis (11) easier.

5. A fixation element according to one of Claims 1 to 4,
**characterised in that**
the peripheral edge(21) of the clamping ring (20) on the end face thereof, directed towards the connection element (17), has rounded peripheral edges (R).

6. A fixation element according to one of Claims 1 to 5,
**characterised in that**
the rod-like connection element (17) is a rigid or plastically deformable rod, or is formed in the manner of a flexible cable.

7. A fixation element according to one of Claims 1 to 6, **characterised in that** the clamping ring (20) is mounted for rotation on the exterior peripheral edge of the cover (18) in such a way that the cover (18) and the clamping ring (20) form a structural unit.

8. An osteosynthetic fixation element, more particularly a pedicle screw (10) or a spine hook, comprising
a) a longitudinal axis (11),
b) a first portion (12) anchorable to the bone,
c) a second portion (13), adjoining the first portion (12) in the direction of the longitudinal axis (11), which comprises a passage (16) defined by two lateral arms (14, 15) for receiving a rod-like connection element (17),
d) a clamping element (30), insertable between the two arms (14, 15) in order to fix the rod-like connection element (17) to effect the clamping thereof within the passage (16) formed in the second portion (13) of the fixation element (10), and comprising
e) a cap (31), adapted for screwing to the second portion (13) of the fixation element (10), the exterior flange (32) of which encloses the two arms (14,15) of the second portion (13) and which is connected to the clamping element (30),
wherein the flange (32) of the cap (31) has an internal thread (33) which corresponds to an external thread (34) formed on the exterior peripheral surface of the two arms (14, 15) of the second pardon (13),
**characterised in that**
the clamping element (30) is mounted on the cover of the cap (31) for rotation relative to the longitudinal axis (11), and
**in that** the end face (35) directed towards the connection element (17) and
cooperating therewith is shaped so as to be plane and extends obliquely to the longitudinal axis (11).

9. A fixation element according to Claim 8,
**characterised in that**
the clamping element (30) is accessible from the exterior through the cover of the cap (31) for the adjustment of a desired rotational position.

10. A fixation element according to Claim 7 or 8,
**characterised in that** the clamping element (30) is a cylinder portion chamfered obliquely on the end face directed towards the rod-like connection element (17) and comprising a smooth peripheral surface.

## Revendications

1. Fixateur pour ostéosynthèse, en particulier vis pédiculaire (10) ou crochet laminaire, comprenant
a) un axe longitudinal (11),
b) une première partie (12) destinée à être ancrée à l'os,
c) une deuxième partie (13), qui est adjacente à la première partie (12) dans la direction de l'axe longitudinal (11) et qui comporte un passage (16), délimité par deux flancs latéraux (14, 15) et destiné à recevoir un élément d'assemblage (17) en forme de tige,
d) un couvercle (18) s'avançant au-delà du bord extérieur des deux flancs (14, 15) et muni d'une partie filetée (19) centrale, destinée à être vissée entre les deux flancs (14, 15), et
e) une bague de serrage (20), qui entoure avec un jeu radial les deux flancs (14, 15) et qui, pour le blocage serré de l'élément d'assemblage (17) en forme de tige à l'intérieur du passage (16) réalisé dans la deuxième partie (13), peut être pressée par le couvercle (18), plus précisément le bord périphérique (21) de celui-ci, contre l'élément d'assemblage (17), sachant que le bord périphérique (21) extérieur orienté vers l'élément d'assemblage (17) s'étend de part en part dans un plan, ou comporte plusieurs évidements, qui sont répartis régulièrement sur le pourtour diamétralement par rapport à l'axe longitudinal (11) et dont la paroi axiale de chacun est adaptée au pourtour associé de l'élément d'assemblage (17) en forme de tige,
**caractérisé en ce que** le plan défini par le bord périphérique (21) extérieur est incliné en oblique (angle α) par rapport à l'axe longitudinal (11).

2. Fixateur selon la revendication 1, **caractérisé en ce que**, dans le fond du passage (16), réalisé dans la deuxième partie (13) du fixateur (10), est logé de manière universellement articulée un élément de support (22) pour l'élément d'assemblage (17) en forme de tige, de telle sorte que l'élément d'assemblage (17) en forme de tige peut pivoter à l'intérieur du plan défini par le passage (16), de même que dans un plan transversal à celui-ci et peut être bloqué au moyen de la bague de serrage (20) dans une position de basculement et de rotation préférée.

3. Fixateur selon la revendication 1, **caractérisé en ce que**, dans le fond du passage (16), réalisé dans la deuxième partie (13) du fixateur (10), est réalisée une élévation (28) en forme d'étrier, par l'intermédiaire de laquelle l'élément d'assemblage (17) en forme de tige peut être amené dans une position de basculement préférée par rapport à l'axe longitudinal (11) du fixateur (10) et peut être bloqué dans cette position au moyen de la bague de serrage (20).

4. Fixateur selon l'une quelconque des revendications 1 à 3, **caractérisé en ce que**, dans le but d'une rotation plus aisée de la bague de serrage (20) autour de l'axe longitudinal (11), la face périphérique extérieure de la bague de serrage (20) est structurée, en particulier est striée.

5. Fixateur selon l'une quelconque des revendications 1 à 4, **caractérisé en ce que** le bord périphérique (21) frontal, orienté vers l'élément d'assemblage (17), de la bague de serrage (20) possède des arêtes (R) arrondies.

6. Fixateur selon l'une quelconque des revendications 1 à 5, **caractérisé en ce que** l'élément d'assemblage (17) en forme de tige est conçu en forme de tige rigide ou plastiquement déformable ou sous la forme d'un câble flexible.

7. Fixateur selon l'une quelconque des revendications 1 à 6, **caractérisé en ce que** la bague de serrage (20) est logée de manière rotative au niveau du bord périphérique extérieur du couvercle (18), de telle sorte que le couvercle (18) et la bague de serrage (20) forment une unité.

8. Fixateur pour ostéosynthèse, en particulier vis pédiculaire (10) ou crochet laminaire, comprenant
a) un axe longitudinal (11),
b) une première partie (12) destinée à être ancrée à l'os,
c) une deuxième partie (13), qui est adjacente à la première partie (12) dans la direction de l'axe longitudinal (11) et qui comporte un passage (16), délimité par deux flancs latéraux (14, 15) et destiné à recevoir un élément d'assemblage (17) en forme de tige,
d) un élément de serrage (30) à poser entre les deux flancs (14, 15) pour bloquer par serrage l'élément d'assemblage (17) en forme de tige à l'intérieur du passage (16) réalisé dans la deuxième partie (13) du fixateur (10), et
e) une calotte (31) à visser sur la deuxième partie (13) du fixateur (10) et dont le rebord (32) extérieur entoure les deux flancs (14, 15) de la deuxième partie (13) et qui est assemblée avec l'élément de serrage (30),
sachant que le rebord (32) de la calotte (31) est muni d'un taraudage (33), qui correspond à un filetage extérieur (34) réalisé sur la face périphérique extérieure des deux flancs (14, 15) de la deuxième partie (13),
**caractérisé en ce que** l'élément de serrage (30) est logé au niveau du couvercle de la calotte (31) de manière rotative par rapport à l'axe longitudinal (11) et **en ce que** la face frontale (35) orientée vers l'élément d'assemblage (17) et coopérant avec celui-ci est plane et s'étend en oblique par rapport à l'axe longitudinal (11).

9. Fixateur selon la revendication 8, **caractérisé en ce que**, pour le réglage d'une position de rotation souhaitée, l'élément de serrage (30) est accessible de l'extérieur à travers le couvercle de la calotte (31).

10. Fixateur selon la revendication 7 ou 8, **caractérisé en ce que** l'élément de serrage (30) est réalisé sous forme de partie cylindrique, qui est chanfreinée au niveau de la face frontale orientée vers l'élément d'assemblage (17) en forme de tige et qui comporte une face périphérique lisse.
